# EUROPEAN PATENT APPLICATION

(11) **EP 1 731 247 A1**
(43) Date of publication of application: **13.12.2006**
(21) Application number: 05447131.3
(22) Date of filing: 07.06.2005
(51) Int. Cl.: B22F 3/11, B22F 3/10

(54) **Titanium, titanium alloy and NiTi foams with high ductility**

(71) Applicant: VLAAMSE INSTELLING VOOR TECHNOLOGISCH ONDERZOEK (VITO), 2400 Mol (BE)
(72) Inventor: Mullens, Steven, 3271 Zichem (BE); Thijs, Ivo, 2400 Mol (BE); Cooymans, Jozef, 2400 Mol (BE); Luyten, Jan, 3294 Molenstede (BE)
(74) Representative: Van Malderen, Joëlle

(57) **Abstract**

The present invention is related to a method for manufacturing a high ductility Ti foam, comprising the steps of
• Preparing a powder suspension of a Ti, NiTi or Ti alloy powder,
• Bring said powder suspension into a desired form to form a green artefact,
• A calcination step wherein said green artefact is calcined, and
• Sintering said artefact,

characterised in that said calcination step comprises a slow heating step wherein said green artefact is heated at a rate lower or equal to 50°C/hour to a temperature between 400°C and 600°C and that the Ti, NiTi or Ti alloy powder has a particle size less than 50 µm.

A high ductility Ti, Ti alloy or NiTi foams with a theoretical density less than 30% van be obtained with such a method.

## Description

### Field of the invention

The present invention is related to high ductility Titanium, titanium alloy or NiTi foams, in particular for use in biomedical or composite structures. The invention relates also to the use of such foams as bone replacement or healing aid.

### State of the art

Titanium and titanium alloys are commonly used for their high strength/weight ratio, their high resistance to corrosion and relatively low elasticity modulus, and their biocompatibility. The mechanical properties of Ti foams are very close to bone properties and therefore, it seems the material of choice for bone replacement or healing aid. However, this is only possible if the ductility of the foams is sufficient. Ductility is more commonly defined as the ability of a material to deform easily upon the application of a tensile force, or as the ability of a material to withstand plastic deformation without rupture, breaking or fracturing. Ductility may also be thought of in terms of bendability and crushability. Ductile materials show large deformation before fracture. The lack of ductility is often termed brittleness.

Titanium is very reactive with a lot of compounds if heated above 300°C. Due to interstitial take-up of N, O, C or H, a brittle foam is obtained, which is not sufficiently strong and ductile to serve as a high-quality bone replacement.

### Aims of the invention

The present invention aims to provide a method for the production of high-ductility Ti, Ti alloy and NiTi foams.

The present invention also aims at providing a high-ductility Ti, Ti alloy and NiTi foams with less than 30 % theoretical density (TD). Preferably, a plastic deformation of 10% without rupture, breaking or fracturing should be achieved.

### Summary of the invention

The present invention concerns a method for manufacturing a high ductility Ti foam, comprising the steps of
- Preparing a powder suspension of a Ti, NiTi or Ti alloy powder,
- Bring said powder suspension into a desired form to form a green artefact,
- A calcination step wherein said green artefact is calcined, and
- Sintering said artefact,
characterised in that said calcination step comprises a slow heating step wherein said green artefact is heated at a rate lower or equal to 50°C/hour to a temperature between 400°C and 600°C and that the Ti, NiTi or Ti alloy powder has a particle size less than 50 *µ*m. Preferably, the heating rate is less than or equal to 25 °C, more preferably 10°C.

Preferably, said calcinations step is performed under an inert atmosphere. Advantageously, said calcination step is performed at a pressure less than 10⁻³ mbar.

The method according to the present invention can further comprise a presintering step comprising heating up the artefact until a temperature of between 900 and 1000°C is reached. It can also comprise a sintering step comprising slow heating of the artefact to a temperature between 1200°C and 1500°C, and keeping said artefact at said temperature for a predetermined period of time.

In a preferred embodiment, the powder comprises less than 0.03 weight % C, less than 0.8 weight % O, less than 0.5 weight % N, less than 0.1 weight % Fe and less than 0.1 weight % Si.

The powder suspension can be brought into the desired form using any method, e.g. a method selected from the group consisting of PU-replica technique, hollow beads technique, and gelcasting.

Another aspect of the present invention concerns a high ductility Ti, Ti alloy or NiTi foams with a theoretical density less than 30%.

### Short description of the drawings

Fig. 1 represents a flowchart of the general method according to the present invention.

Fig. 2 represents the effects of the sand blasting step on the surface of a Ti foam artefact obtained according to the present invention. The artefact surface is shown before (fig. 2 a) and after (fig. 2 b) the sand blasting operation.

Fig. 3 shows a typical pore size distribution of a Ti foam structure obtained by gelcasting according to the present invention.

Fig. 4 shows the relation between stress and deformation for several sintering temperatures for a foam according to the present invention.

Fig. 5 shows a Ti foam obtained with the PU replica technique, with (5a) and without (5b) TiH₂.

Fig. 6 shows the shape memory effect of a NiTi foam structure obtained according to the present invention.

### Detailed description of the invention

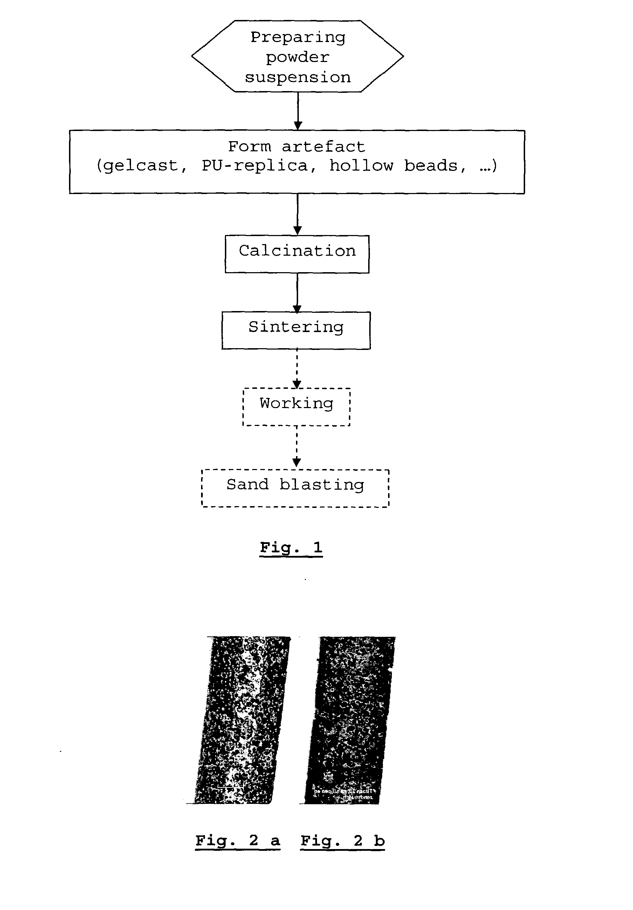

A more detailed description of the present invention is presented by means of examples and figures. The general process according to the present invention is given in figure 1.

The powder suspension is prepared by mixing water and Ti powder (or powder of a Ti alloy such as Ti6Al4V and NiTi, with a powder volume of between 20 and 50%) with additives such as gelling aid or other, depending on the method selected for forming the artefact. The skilled person is well acquainted with these additives and can easily prepare a suitable powder suspension according to this first step of the process, suitable for the chosen forming process. Also, the viscosity of the suspension can be easily adapted to the desired range for the selected forming process. Examples of gelling aids and other additives, which can be used for obtaining a stable Ti suspension according to the known methods as presented in the forming step of figure 1, are given next:
**Gelling aids:**
   - Gelatine
   - Agarose
   - Other hydrocolloids or biobinders (pectins, starch, xanthane, carragene, ...)
**Other additives**
   • Cellulose-derived (e.g. hydroxypropyl methylcellulose)
   • Alginate and its derivatives
   • Dispersion agents (such as targon™ 1128, DarvanC™, ...)
   • Foaming agents (for gelcasting)
   • Anti-foaming agents (for PU replica technique and hollow beads technique)

In order to obtain a suitable suspension for the process according to the present invention, the particle size of the Ti powder should be less than 50 *µ*m, the particle shape should be spherical or irregular (depending on the milling method) and of high purity, with less than 0.03 weight % C, less than 0.8 weight % O, less than 0.5 weight % N, less than 0.1 weight % H, less than 0.1 weight % Fe and less than 0.1 weight % Si.

For the PU replica method, the powder suspension preferably contains 10 to 50 volume % Ti powder which can advantageously be replaced by a specific fraction of TiH₂ with a particle size smaller than 10 *µ*m. This bimodal particle size distribution provides a better wetting of the PU foam. Also, the dehydrogenation of TiH₂ can have a positive effect on the amount of impurities in the Ti foam after calcination.

In the second step, the suspension is formed to obtain the desired structure of the artefact. Depending on the desired structure, different techniques can be used.

Gel casting with hydrocolloids or biobinders as gelling agent produces a highly porous structure (70-90% TD), open pores with pore dimensions between 1 and 500 *µ*m (depending on the powder suspension and process parameters such as type and quantity of foaming agent, viscosity of the suspension, foaming procedure, ...) and high interconnectivity.

The PU replica technique can produce Ti foams with a pore size between 300 *µ*m and several mm. Open pores are obtained. Depending on the cell structure of the polymer foam used, a porous Ti structure with a certain cell structure can be obtained.

To obtain a mixed open/closed porosity, one can use the hollow spheres method, wherein organic spherical structures (e.g. vegetable seeds) are coated with the suspension. The coated spheres are then attached to one another by ordering the coated spheres to obtain the desired structure and apply a second coating step with the powder suspension. After calcination, the organic spherical structures being burnt out, a mixed open/closed structure can be obtained. The structure is closed where the spherical structures were positioned and is open in between said spherical structures.

The calcination step is preferably executed in two steps. In the first step, a slow heating (maximum 50°C per hour) until a temperature of about 400 to 600 °C is obtained (preferably less than 500°C). Advantageously, this is done under inert atmosphere such as argon or even better at low pressure (10⁻³ mbar or less). During this first step, most of the organic material present will be burnt away. In case TiH₂ is used, the foam should be kept at 650°C during 24 hours in a high vacuum to remove the hydrogen.

The second step of the calcination step is a presintering step until a temperature of 900 to 1000°C is reached. This results in a structure where the Ti powder particles are already starting to sinter together, allowing handling of the structure. Preferably, this step is performed under a vacuum of at least 10⁻⁵ mbar on a Mo plate.

Next, the obtained structure is sintered by placing it on a Mo strip or rolls, allowing shrinking of the structure. Again, a high vacuum of more than 10⁻⁵ mbar is used. If necessary, the Ti foam can be co-sintered to a dense Ti structure.

Optionally, a working step can be used in case it is necessary to remove e.g. reaction zones situated at the contact points with the Mo support material or when other structure corrections (e.g. sawing) are desired.

Also, a sand blasting operation can be useful to open up the pores at the surface of the artefact. The result of such a sand blasting operation can be seen in figure 2.

### Examples

### 1. Ti foam structure obtained by gelcasting:

A suspension is prepared using 300 g Ti Cerac 99,5% pyre (-325 mesh), 201 g H₂O, 6.4 g Agar (3,18 % on H₂O), 6 g Tergitol TMN10 (2 % on Ti), 3 g Triton (1 % on Ti) and 0.36 g Ammonium Alginate (0,18 % on H₂O). It is mixed during 6 minutes at 70°C to obtain a fluid foam. The foam is cast into a mould and cooled down until the structure is gellified. After demoulding, the structure is dried at atmospheric pressure and room temperature.

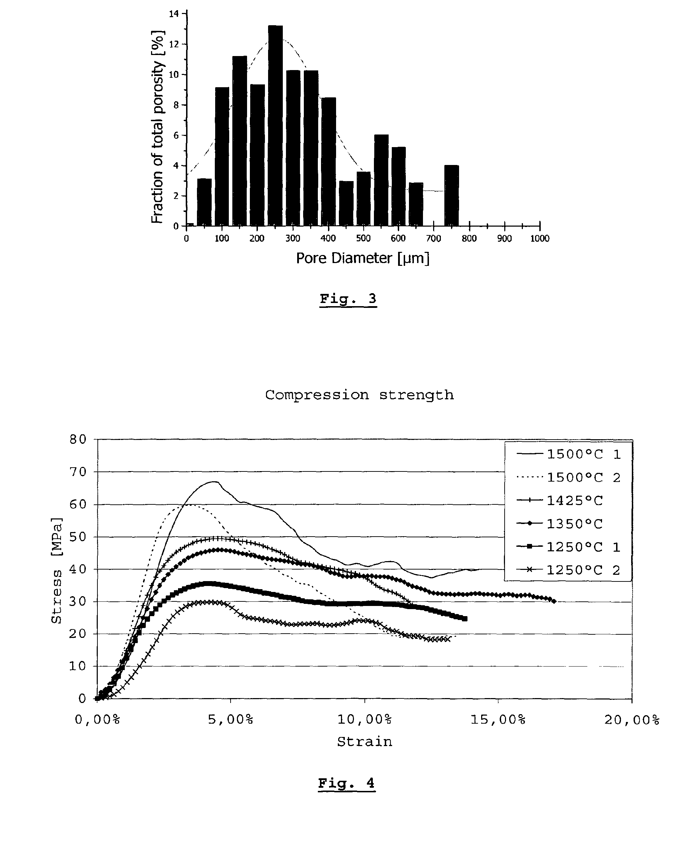

The structure is then calcined (10⁻³ mbar, 25°C/h until 500°C, 2 hours isotherm, in the presence of Zr metal) and sintered (10⁻⁵ mbar, 5°C/min to 1200-1500°C; 2h isotherm). An open-structured Ti foam with pore size between 100 and 400 *µ*m is obtained. A typical pore size distribution is shown in figure 3. A stress-strain curve is presented in figure 4. For 1500°C and 1250°C two different measurements with different loads were performed.

Properties of the obtainable structures include (depending on sintering temperature):

| E-modulus (GPa) | |
|---|---|
| 1500°C | 2.5 |
| 1425°C | 2.1 |
| 1350°C | 1.8 |
| 1250°C | 1.5 |

| Specific surface (m²/g) | |
|---|---|
| 1500°C | 0.7 |
| 1250°C | 1.3 |

| Porosity (%TD) | |
|---|---|
| 1500°C | 70% |
| 1250°C | 75% |

### 2. Ti foam via replica method with TiH₂

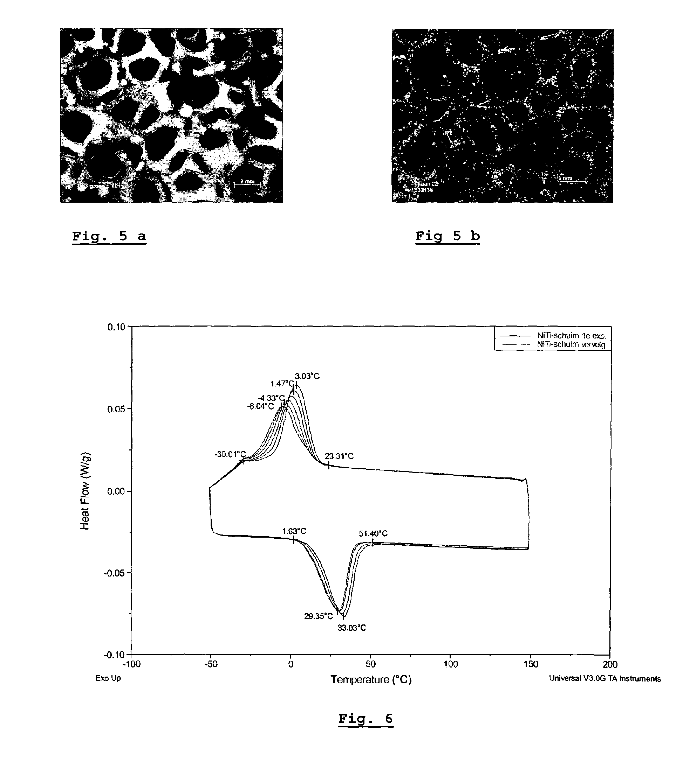

56 g fine Ti Cerac T-1191 powder, 62.75 g H₂O, 2.82 g gelatine (4,5 % on H₂O), 0.125 g Benecel MP874 Herculus (0.2% on H₂O), 3.18 g Targon 1128 and 0.5 g antifoam are mixed. A PU polymer foam is submerged in the suspension and the excess suspension is removed. The mechanical properties of the end product are highly influenced by the wetting of the PU polymer by the suspension. Use of TiH₂ positively influences the wetting of the PU polymer, as can be seen in figure 5 a (with TiH₂) and b (without TiH₂).

After drying, the structure is calcined and sintered. The mechanical and structural properties are very close to those of human bone:

| | Bone | Ti-foam |
|---|---|---|
| Porosity (%) | 82.7 | 86.6 |
| Pore interconnectivity (%) | 100 | 100 |
| Connectivity density (1/mm | 4.02 | 27.90 |
| Pore size | | |
| % pores 100-800µm | 95.8 | 99.5 |
| Largest pore (mm) | 1.5 | 1.38 |
| Permeability (mm) | 6.46 E-08 | 3.04 E-08 |
| Specific surface area (/mm) | 2.99 | 4.25 |
| Degree of anisotropy | 2.28 | 1.39 |
| Apparent stiffness (MPa) | 287 | 315 |

### 3. NiTi Foams

NiTi can also be used for producing foams. An example is a NiTi foam created with the gelcasting technique.

Differential Scanning Calorimetry (DSC, TA Instruments 2920) was used to measure the shape memory effect of such a foam. The transition temperature is clear from figure 6. These materials are very promising for biomedical applications as the transitions occur between about 0 and 40°C.

## Claims

1. A method for manufacturing a high ductility Ti foam, comprising the steps of
• Preparing a powder suspension of a Ti, NiTi or Ti alloy powder,
• Bring said powder suspension into a desired form to form a green artefact,
• A calcination step wherein said green artefact is calcined, and
• Sintering said artefact,
**Characterised in that** said calcination step comprises a slow heating step wherein said green artefact is heated at a rate lower or equal to 50°C/hour to a temperature between 400°C and 600°C and that the Ti, NiTi or Ti alloy powder has a particle size less than 50 *µ*m.

2. The method according to claim 1, wherein said calcinations step is performed under an inert atmosphere.

3. The method according to claim 1 or 2, wherein said calcination step is performed at a pressure less than 10⁻³ mbar.

4. The method according to any of the claims 1 to 3, further comprising a presintering step comprising heating up the artefact until a temperature of between 900 and 1000°C is reached.

5. The method according to any of the claims 1 to 4, further comprising a sintering step comprising slow heating of the artefact to a temperature between 1200°C and 1500°C, and keeping said artefact at said temperature for a predetermined period of time.

6. The method according to any of the claims 1 to 5, wherein the powder comprises less than 0.03 weight % C, less than 0.8 weight % O, less than 0.5 weight % N, less than 0.1 weight % Fe and less than 0.1 weight % Si.

7. The method according to any of the claims 1 to 6, wherein the powder suspension is brought into the desired form using a method selected from the group consisting of PU-replica technique, hollow beads technique, and gelcasting.

8. A high ductility Ti, Ti alloy or NiTi foams with a theoretical density less than 30%.
